(19) Europäisches Patentamt
European Patent Office
Office européen des brevets

(11) **EP 3 996 663 B1**

(12) **EUROPEAN PATENT SPECIFICATION**

(45) Date of publication and mention
of the grant of the patent:
**07.08.2024 Bulletin 2024/32**

(51) International Patent Classification (IPC):
***A61K 8/34*** *(2006.01)* ***A61K 8/49*** *(2006.01)*
***A61Q 5/00*** *(2006.01)*

(21) Application number: **20733316.2**

(22) Date of filing: **22.06.2020**

(52) Cooperative Patent Classification (CPC):
**A61Q 5/006; A61K 8/0241; A61K 8/342;
A61K 8/736; A61K 8/8111;** A61K 2800/412

(86) International application number:
**PCT/EP2020/067359**

(87) International publication number:
**WO 2021/004770 (14.01.2021 Gazette 2021/02)**

(54) **HAIR CARE COMPOSITION COMPRISING ANTIDANDRUFF AGENT**

HAARPFLEGEZUSAMMENSETZUNG MIT EINEM ANTISCHUPPENMITTEL

COMPOSITION DE SOINS CAPILLAIRES COMPRENANT UN AGENT ANTIPELLICULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.07.2019 PCT/CN2019/095311
23.08.2019 EP 19193269**

(43) Date of publication of application:
**18.05.2022 Bulletin 2022/20**

(73) Proprietors:
• **Unilever IP Holdings B.V.**
**3013 AL Rotterdam (NL)**
Designated Contracting States:
**AL AT BE BG CH CZ DK EE ES FI FR GR HR HU
IS LI LT LU LV MC MK NL NO PL PT RO SE SI SK
SM**
• **UNILEVER GLOBAL IP LIMITED**
**Wirral**
**Merseyside CH62 4ZD (GB)**
Designated Contracting States:
**CY DE GB IE IT MT RS TR**

(72) Inventors:
• **MERRINGTON, James**
**Wirral Merseyside CH63 3JW (GB)**

• **PAN, Xiaoyun**
**Shanghai, 200335 (CN)**
• **TANG, Xuezhi**
**Shanghai, 200335 (CN)**
• **WOOD, Ian, Geoffrey**
**Wirral, Merseyside CH63 3JW (GB)**

(74) Representative: **Mathai, Neenu Grace**
**Unilever Patent Group**
**Bronland 14**
**6708 WH Wageningen (NL)**

(56) References cited:
**WO-A1-2004/082649** **WO-A1-2014/124066**
**WO-A1-2018/033328** **WO-A1-96/22074**
**US-A1- 2013 089 586** **US-B1- 6 355 261**

• **MANGESH B. INARKAR ET AL: "Extraction and
Characterization of Sugarcane Peel Wax", ISRN
AGRONOMY, vol. 2012, 1 January 2012
(2012-01-01), pages 1 - 6, XP055663844, DOI:
10.5402/2012/340158**
• **FREELAND M S ET AL: "Cationic Guar Gum",
COSMETICS & TOILETRIES, ALLURED
PUBLISHING CORP, US, vol. 99, no. 6, 1 June 1984
(1984-06-01), pages 83 - 87, XP009105443, ISSN:
0361-4387**

**Description**

**Field of the Invention**

The present invention relates to hair care compositions that comprise an antidandruff agent, more particularly piroctone olamine.

**Background of the Invention**

The efficacy of an antidandruff shampoo or conditioner depends largely on the amount of the antidandruff agent deposited on the scalp and hair. Usually, at the point of use, the contact time of hair/scalp with any wash-off haircare product is very short e.g., 10 to 120 seconds after which the composition gets washed off, so the antidandruff agent may not get enough time for deposition. Therefore, in the limited time of contact, it is desirable to deposit as much of the agent as possible. However, deposition of any active ingredient, especially antidandruff agent, through a wash-off haircare composition presents a technical problem because some amount of the particles of the antidandruff agent tend to wash off rather than deposit. To offset the loss, an increase in the amount of the antidandruff agent is a possible solution but it is neither technically sound nor economically viable.

Piroctone olamine (Octopirox®) is a widely used antidandruff agent. However, insufficient deposition remains a technical problem. Another problem is instability in shampoos.

US20040213751 A1 (P&G) discloses a haircare composition comprising pyrithione and a zinc-containing layered material which provides an augmentation factor greater than 1.

WO14124066 A1 (P&G) discloses hair care compositions comprising cationic polymers and anionic particulates for improved deposition of pyrithione.

WO2008101546 (Rovi) discloses a cosmetic preparation with an active ingredient for the protection or treatment of the skin and / or the hair and with a carrier material in or to which the active ingredient is bound or associated with the active ingredient, wherein the carrier material comprises a proportion of chitosan. The active substance is bound with or is associated with a carrier material is a fraction having chitosan and a proportion of polylactide, polyglycolide and / or polylactide glycolide or derivatives thereof, and wherein the carrier material is in the form of particles having an average particle size of 10 to 1000 nm.

WO10105922 A1 [Unilever] discloses a particle comprising a waxy solid and a polymeric deposition aid having no overall cationic charge and a method to prepare such particle.

Our co-pending application EP19154998 (Unilever) discloses composite particles comprising a photolabile antidandruff agent and an organic UV filter whose melting point is from 30°C to 105°C, characterized in that said composite particles comprise a cationic polymer having weight average molecular weight of 1000 Da to 10000000 Da.

WO9823258 A1 (Unilever) discloses a shampoo containing piroctone olamine and 0.1 to 5 % by weight polyethylenimine to enhance the deposition of piroctone olamine.

**Summary of the Invention**

It has been determined that at least some of the problems of prior art can be solved by way of the present invention.

In accordance with a first aspect is disclosed a composite particle comprising:

(i) an antidandruff agent;
(ii) wax or a wax-like substance in which said antidandruff agent is soluble or dispersible, where melting point of said wax and said substance is 30°C to 105°C and where said substance is not a UV absorbing sunscreen; and,
(iii) a cationic polymer having weight average molecular weight of $10^3$ Da to $10^7$ Da, where said composite particle has particle size 0.1 to 1000 microns and where said wax-like substance is a C13 to 35 fatty alcohol; where said wax is at least one of beeswax, Chinese wax, lanolin, shellac wax, spermaceti, bayberry wax, candelilla wax, carnauba wax, castor wax, esparto wax, Japan wax, ouricury wax, rice bran wax, soy wax, tallow tree wax, ceresin wax, montan wax, ozocerite or peat wax.

We have determined that particles of the invention not only serve to deposit more of the antidandruff agent on the scalp but also stabilise the antidandruff agent, especially piroctone olamine, in a haircare product, such as a shampoo composition. Such agents may lose their efficacy over a period presumably due to presence of surfactants, polymers and other ingredients which may degrade or destabilize the antidandruff agent.

In accordance with a second aspect, disclosed is a method of preparing a composition as claimed in claim 1 comprising a step of heating and agitating an aqueous slurry comprising said wax or wax-like substance and said antidandruff agent, followed by a step of adding said cationic polymer to said slurry and further heating said slurry for 5 to 60 minutes at 30 to 100 °C.

In accordance with a third aspect is disclosed a hair care product comprising a composition of the first aspect.

## Detailed Description of the Invention

For the avoidance of doubt, any feature of one aspect of the present invention may be utilized in any other aspect of the invention. The word "comprising" is intended to mean "including" but not necessarily "consisting of or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. As used herein, the indefinite article "a" or "an" and its corresponding definite article "the" means at least one, or one or more, unless specified otherwise. The various features of the present invention referred to in individual sections above apply, as appropriate, to other sections *mutatis mutandis.* Consequently, features specified in one section may be combined with features specified in other sections as appropriate. Any section headings are added for convenience only and are not intended to limit the disclosure in any way.

By "haircare product" as used herein, is meant to include a composition for topical application to hair or scalp of mammals, especially humans. By topical is meant that the product is applied to the external surface of the body. In the present invention this is achieved by applying the haircare product to hair or scalp. Such a product may be generally classified as leave-on or rinse off, and includes any product applied for improving the appearance, cleansing, odor control or general aesthetics of scalp and hair. The haircare product of the present invention is preferably a leave-on product. Alternatively, the haircare product of the present invention is a wash-off composition. A haircare product in accordance with the present invention is preferably a shampoo, hair conditioner, hair cream, hair colour, hair serum, mousse, hair gel or hair oil.

In a first aspect, the composite particle of the invention comprises:

(i) an antidandruff agent;
(ii) wax or a wax-like substance in which said antidandruff agent is soluble or dispersible, where melting point of said wax and said substance is 30°C to 105°C and where said substance is not a UV absorbing sunscreen; and,
(iii) a cationic polymer having weight average molecular weight of $10^3$ Da to $10^7$ Da, where said composite particle has particle size 0.1 to 1000 microns and where said wax-like substance is a C13 to 35 fatty alcohol; where said wax is at
least one of beeswax, Chinese wax, lanolin, shellac wax, spermaceti, bayberry wax, candelilla wax, carnauba wax, castor wax, esparto wax, Japan wax, ouricury wax, rice bran wax, soy wax, tallow tree wax, ceresin wax, montan wax, ozocerite or peat wax.

The term particles means particles ranging in size from 0.1 to 1000 $\mu$m, preferably from 0.1 to 100 $\mu$m, most preferably from 1 to 50 $\mu$m. Preferably the composition of the invention is powdery.

Alternatively, the composition of the invention, i.e., the particles is powdery, preferably a freeze-dried powder. The size may be measured for example, by laser diffraction using a system (such as a Mastersizer™ 2000 available from Malvern Instruments Ltd). It is preferred that ratio of the amount of the antidandruff agent to the amount of said wax or wax-like substance in said microparticles is from 1:0.01 to 1:1000 parts by weight.

It is preferred that the microparticles comprise 0.1 to 80 wt% antidandruff agent, 18 to 95 wt% of the wax or wax-like substance and 0.5 to 10 wt% cationic polymer.

## Wax or wax-like substance

The composition of this invention comprises a wax or a wax-like substance in which the antidandruff agent is soluble or dispersible, where melting point of the wax and the substance is 30°C to 105°C. The wax-like substance is not a UV absorbing sunscreen.

Melting point refers to the temperature at which the solid and liquid forms of a pure substance can exist in equilibrium.

The wax of the invention is at least one of beeswax, Chinese wax, lanolin, shellac wax, spermaceti, bayberry wax, candelilla wax, carnauba wax, castor wax, esparto wax, Japan wax, ouricury wax, rice bran wax, soy wax, tallow tree wax, ceresin wax, montan wax, ozocerite or peat wax. Such waxes are often selected from hydrocarbon waxes and ester waxes, that can be derived from natural sources or synthesized. Suitable hydrocarbon waxes include mineral wax, microcrystalline wax, Montana wax, and low molecular weight polyethylene, such as from 300 to 600 daltons. Suitable ester waxes can be derived from unsaturated natural oils, such as plant-originating triglyceride oils by hydrogenation and optionally dehydroxylation (where the substituent contains at least one hydroxyl group as in castor oil). Suitable ester waxes include caster wax, candelilla wax, carnauba wax, beeswax and spermeceti wax. Natural waxes such as beeswax include a range of different chemical classes. Synthetic esters often comprise aliphatic monoesters containing at least 30 carbons, and indeed may be isolated natural products such as beeswax, or be derived from them or be the same compounds.

The wax-like substance of the present invention is a C13-35 fatty alcohol.

One or a blend of fatty alcohols can be used. Preferred fatty alcohols include cetostearyl alcohol, cetyl alcohol, stearyl alcohol, eicosyl alcohol and behenyl alcohol. Commercial fatty alcohols, though nominally and predominantly one specified alcohol often comprise a minor fraction, such as up to 5 or 6% by weight in total, of homologues differing by 2, 4 or even 6 carbons.

The wax and the wax-like substance is not a UV absorbing sunscreen. For example, some sunscreens are listed below: 2-hydroxy-4-methoxybenzophenone (also named as Benzophenone-3 CAS: 131-57-7, MP 62 to 64°C), 2,2-dihydroxy-4-methoxybenzophenone (CAS: 131-53-3, MP 73 to 75°C), butylmethoxydibenzoylmethane (CAS: 70356-09-1, MP 81 to 84°C), bis-ethylhexyloxyphenol methoxyphenyl triazine (Tinosorb S, CAS: 187393-00-6, MP 83-85°C), Menthyl anthranilate (CAS: 134-09-8, MP 62.5-63.5°C), 4-Methylbenzylidene camphor (Enzacamene) (CAS: 36861-47-9, MP 66 to 69°C), Benzophenone-7 (5-chloro-2-hydroxybenzophenone) (CAS: 85-19-8, MP 96 to 98°C), Benzophenone-8 (dioxybenzone) (CAS: 131-53-3, MP 68°C), Benzophenone-10 (mexenone, 2-hydroxy-4-methoxy-4'-methyl-benzophenone, CAS: 1641-17-4, MP 99 to 102°C), Benzophenone-12 (octabenzone) (CAS: 1843-05-6, MP 47 to 49°C).

## Antidandruff agent

The composition of the invention, i.e., the microparticles, preferably comprise from 0.1 to 80 wt.%, preferably from 0.2 to 40 wt.%, more preferably from 0.25 to 15 wt.% of the antidandruff agent. Antidandruff agents are compounds that are active against dandruff and are typically antimicrobial agents, preferably antifungal agents. Antidandruff agents typically display a minimum inhibitory concentration of about 50 mg/ml or less against *Malassezia.*

It is preferred that the anti-dandruff agent is piroctone olamine, climbazole, selenium sulphide, zinc pyrithione, or zinc sulfate.

## Cationic polymer

The compositions of the invention also comprise a cationic polymer. Preferably the polymer is polyamine, polyvinylpyrrolidone, polylysine, protamine, trimethylammonioethyl (meth)acrylate homopolymer and copolymer, acrylamidopropyl trimethylammonium halide homopolymer and copolymer, dialkyldiallylammonium halide homopolymer and copolymer, chitosan or derivatized chitosan, cellulose derivatives comprising trimethyl ammonium substituted epoxide, starch hydroxypropyl trimethyl ammonium halide, polyethyleneimines or polycondensates containing diquaternary ammonium or polyquaternary ammonium repeating units.

The term cationic polymer is used to distinguish such polymers from anionic, i.e., negatively charged polymers as well as from non-ionic polymers, i.e., polymers devoid of any charge.

It is preferred that molecular weight of the cationic polymer is 30,000 to 1,000,000 Daltons, more preferably from 70,000 to 600,000 Daltons, and still even more preferably from 150,000 to 400,000 Daltons.

Zeta potential is the charge that develops at the interface between a solid surface and its liquid medium. This potential, which is measured in Millivolts, may arise by any of several mechanisms. Among these are the dissociation of ionogenic groups in the particle surface and the differential adsorption of solution ions into the surface region. The net charge at the particle surface affects the ion distribution in the nearby region, increasing the concentration of counterions close to the surface. Thus, an electrical double layer is formed in the region of the particle-liquid interface. Zeta potential is therefore a function of the surface charge of the particle, any adsorbed layer at the interface, and the nature and composition of the surrounding suspension medium. It can be experimentally determined and, because it reflects the effective charge on the particles and is therefore related to the electrostatic repulsion between them, the zeta potential has proven to be extremely relevant to the practical study and control of colloidal stability and flocculation processes. A variety of methods and apparatus are available for its measurement with a reasonable degree of precision. For example, the zeta potential of the particles is measured using a Malvern Nano ZS90 apparatus, in DI water at a solid content of 50 ppm and pH of 7 at 25°C.

It is preferred that zeta potential of the cationic polymer is from +10 to +100 mV. It is particularly preferred that the polymer is chitosan. It is preferred that the chitosan comprises a chitosan component and an anion. It is preferred that the chitosan is a salt of chitosan and an amino acid. Preferably the amino acid comprises glutamine, glutamic acid, histidine, leucine, lysine, serine, threonine, arginine or a mixture thereof, more preferably arginine.

Preferably, the chitosan comprises at least 5%, more preferably at least 10% of protonated primary amino group by mole of the total amount of primary amino group and protonated primary amino group.

It is preferred that degree of deacetylation of chitosan is at least 65%, more preferably from 70 to 95%, even more preferably from 72 to 90% and most preferably from 75 to 85%.

It is preferred that ratio of the antidandruff agent to the cationic polymer in said microparticles is from 1:0.01 to 1:1000 parts by weight.

It is preferred that the compositions of the invention, i.e., particles, comprise a co-solvent in which said anti-dandruff agent is soluble or dispersible, where said co-solvent is a ketone and where said co-solvent is neither wax nor a waxy-like substance. This co-solvent is in addition to the wax or wax-like substance.

It is preferred said co-solvent is a ketone selected from 2-hexanone or 2-octanone or damascone.

More preferably this ketone is damascone.

Method of preparing the composite particles

In accordance with a second aspect is disclosed a method of preparing a composition of the first aspect comprising a step of heating and agitating an aqueous slurry comprising said wax or wax-like substance and said antidandruff agent, followed by a step of adding said cationic polymer to said slurry and further heating said slurry for 5 to 60 minutes at 30 to 100 °C to get the composite particle or a slurry of the composite particle.

Hair care composition

In accordance with a third aspect is disclosed a hair care product comprising a composition of the first aspect. More preferably the haircare product comprises an amount of the composition (i.e., microparticles) such that the total amount of the antidandruff agent in said haircare product is from 0.01 to 5.0 wt%.

Further preferably the haircare product is a shampoo, hair conditioner, hair cream, hair colour, hair serum, mousse, hair gel or hair oil.

In addition to the antidandruff agent that is present in the form of the composite particles, the haircare compositions in accordance with this invention may also comprise additional antidandruff agent which may, for example, be the same as the one contained inside the microparticles. Whenever present, the haircare compositions of the invention preferably comprise 0.05 to 5 wt% of additional antidandruff agent. The additional antidandruff agent is preferably selected from azoles, Octopirox® (piroctone olamine), selenium sulfide, salicylic acid and combinations thereof. Azoles include ketoconazole and climbazole, preferably climbazole.

The haircare compositions of the invention may further comprise a zinc salt. The additional zinc salt may suitably be selected from zinc salts of organic acids, zinc salts of inorganic acids, zinc oxides, zinc hydroxides or a mixture thereof.

Examples of preferred zinc salts include zinc oxide, zinc pyrrolidone carboxylic acid, zinc citrate, zinc carbonate, zinc chloride, zinc sulphate, zinc glycinate, zinc acetate, zinc lactate, and mixtures thereof. When present, it is preferred that the haircare compositions of the invention comprise 0.1 to 5 wt%, preferably 0.2 to 3 wt%, more preferably from 0.25 to 2.5 wt% of the salt based on the total weight of the composition. The haircare compositions of the present invention comprises a surfactant selected from the group consisting of anionic surfactants, nonionic surfactants, zwitterionic surfactants and mixtures thereof. The nature, type, amount and specific combinations that may be used depend on the formulation of the composition and would largely depend on whether it is a shampoo or a conditioner or a conditioning shampoo.

Preferably, the haircare product of the invention is a shampoo. Preferably it comprises a surfactant which is sodium lauryl sulphate, sodium lauryl ether sulphate, sodium lauryl ether sulphosuccinate, ammonium lauryl sulphate, ammonium lauryl ether sulphate, sodium cocoyl isethionate and lauryl ether carboxylic acid, coco betaine, cocamidopropyl betaine, sodium cocoamphoacetate or a mixture thereof.

Preferably, the haircare product of the present invention comprises from 1 to 50%, preferably from 2 to 40%, more preferably from 4 to 25% total surfactant.

It is further preferred that the haircare product of the invention comprises a cosmetic ingredient. Preferably the cosmetic ingredient is selected from the group consisting of a silicone, an antibacterial agent other than antidandruff agents, a foam booster, a perfume, encapsulates (for example encapsulated fragrance) a dye, a colouring agent, a pigment, a preservative, a thickener, a protein, a phosphate ester, a buffering agent, a pH adjusting agent, a pearlescer (for example; mica, titanium dioxide, titanium dioxide coated mica, ethylene glycol distearate (INCI glycol distearate)) and/or opacifier, a viscosity modifier, an emollient, a sunscreen, an emulsifier, a sensate active (for example menthol and menthol derivatives), vitamins, mineral oils, essential oils, lipids, natural actives, glycerin, natural hair nutrients such as botanical extracts, fruit extracts, sugar derivatives and amino acids, microcrystalline cellulose and mixtures thereof.

Preferably, the haircare product of the present invention includes from 0.01 to 20 wt% of the at least one cosmetic ingredient, more preferably from 0.05 to 10 wt%, still more preferably from 0.075 to 7.5 wt% and most preferably, from 0.1 to 5 wt% of the at least one cosmetic ingredient, by weight of the total product.

The haircare product of the present invention may also comprise synergistic antimicrobial compounds that give synergistic antimicrobial benefit when used in combination with the antidandruff active (for example zinc pyrithione) to enhance its properties and further inhibit the growth of Malassezia furfur. Non-limiting examples of these compounds include compounds having alcoholic groups (e.g. honokiol, magnolol or paeonol), Piperazines and a phenolic compound found in natural plant extract viz. thymol and terpeniol.

The haircare product may additionally comprise a vitamin B3 compound. The preferred vitamin B3 compound is niacinamide.

Niacinamide is known for secretion of AMPs (Anti-Microbial Proteins) from keratinocytes. The AMPs thus secreted provides for improving immunity of e.g. the scalp. Thus with the use of niacinamide, the anti-dandruff efficacy can be enhanced not just through anti-fungal activity but by boosting the scalp's own protection shield against germs, through use of niacinamide. This combination could provide further long-lasting protection e.g. up to 24 hours of protection against germs.

When present, it is preferred that the haircare compositions of the invention comprise 0.1 to 5% niacinamide, more preferably 0.5 to 5%, furthermore preferably 0.5 to 3%, and optimally 1.0 to 3.0% by weight of the composition.

Silicone

It is preferred that the haircare product of the invention comprises silicone.

For example, the compositions of the invention may contain emulsified droplets of a silicone conditioning agent for enhancing conditioning performance.

Suitable silicones include polydiorganosiloxanes, polydimethylsiloxanes which have the CTFA designation dimethicone. In addition, suitable for use in the compositions of the invention (particularly shampoos and conditioners) are polydimethyl siloxanes having hydroxyl end groups, which have the CTFA designation dimethiconol.

Preferably the viscosity of the emulsified silicone is at least 10,000 cst at 25 °C, the viscosity of the silicone is preferably at least 60,000 cst, most preferably at least 500,000 cst, ideally at least 1,000,000 cst. Preferably the viscosity does not exceed $10^9$ cst for ease of formulation.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation. A further preferred class of silicones for inclusion in shampoos and conditioners are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

It is preferred that the total amount of silicone is 0.01 to 10 %wt, more preferably 0.1 to 5 wt% and most preferably 0.5 to 3 wt%.

pH of Compositions

It is preferred that pH of the haircare product of the present invention is preferably from 3 to 7, more preferably 4 to 7, even more preferably 4 to 6.5, most preferably from 4.2 to 6.5.

Shampoos

When the haircare product of the invention is a shampoo, it is generally aqueous, i.e. they have water or an aqueous solution or a lyotropic liquid crystalline phase as their major component.

Suitably, the shampoo composition comprises 50 to 98%, preferably from 60 to 92% water.

Preferably the shampoo composition comprises one or more cationic polymers for conditioning the hair.

Suitable cationic polymers include homopolymers which are cationically substituted or may be formed from two or more types of monomers. The weight average ($M_w$) molecular weight of the polymers will generally be between 100000 and 3 Million Daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus, when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general, secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable (non-limiting examples of) cationic polymers include:

- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides (as described in WO95/22311).

Other cationic polymers that can be used include cationic polysaccharide polymers, such as cationic cellulose derivatives, cationic starch derivatives, and cationic guar gum derivatives.

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14 and JAGUAR C17.

Mixtures of any of the above cationic polymers may be used.

It is preferred that the haircare product of the invention comprises 0.01 to 5%, preferably from 0.02 to 1%, more preferably from 0.05 to 0.8% cationic polymer.

The haircare product of the invention may additionally comprise a cationic deposition polymer which is a cationic polygalactomannans having an average molecular weight ($M_w$) of from 1 million to 2.2 million g/mol and a cationic degree of substitution of from 0.13 to 0.3.

The polygalactomannans are polysaccharides composed principally of galactose and mannose units and are usually found in the endosperm material of seeds from leguminous plants such as guar, locust bean, honey locust, flame tree, and other members of the *Leguminosae* family. Polygalactomannans are composed of a backbone of 1→4-linked β-D-mannopyranosyl main chain units (also termed mannoside units or residues) with recurring 1→6-linked α-D-galactosyl side groups (also termed galactoside units or residues) branching from the number 6 carbon atom of a mannopyranose residue in the polymer backbone. The polygalactomannans of the different *Leguminosae* species differ from one another in the frequency of the occurrence of the galactoside side units branching from the polymannoside backbone. The mannoside and galactoside units are generically referred to herein as glycoside units or residues. The average ratio of mannoside to galactoside units in the polygalactomannan contained in guar gum (hereinafter termed "guar") is approximately 2:1.

Suitable cationic polygalactomannans include guar and hydroxyalkyl guar (for example hydroxyethyl guar or hydroxypropyl guar), that has been cationically modified by chemical reaction with one or more derivatizing agents.

In a typical composition the amount of cationic polygalactomannans will generally range from about 0.05 to 1%, preferably from 0.1 to 0.8%, more preferably 0.2 to 0.6% by weight of the composition.

The haircare product of the invention may additionally comprise an anionic polymeric rheology modifier such as a carboxylic acid polymer.

The term "carboxylic acid polymer" in the context of this invention generally denotes a homopolymer or copolymer obtained from the polymerization of ethylenically unsaturated monomers containing pendant carboxylic acid groups (hereinafter termed "carboxylic monomers").

Suitable carboxylic monomers generally have one or two carboxylic acid groups, one carbon to carbon double bond and contain a total of from 3 to about 10 carbon atoms, more preferably from 3 to about 5 carbon atoms.

Specific examples of suitable carboxylic monomers include $\alpha$-$\beta$-unsaturated monocarboxylic acids such as acrylic acid, methacrylic acid and crotonic acid; and $\alpha$-$\beta$-unsaturated dicarboxylic acids such as itaconic acid, fumaric acid, maleic acid and aconitic acid. Salts, esters or anhydrides of the $\alpha$-$\beta$-unsaturated mono- or dicarboxylic acids described above may also be used. Examples include half esters of $\alpha$-$\beta$-unsaturated dicarboxylic acids with $C_{1-4}$ alkanols, such as monomethyl fumarate; cyclic anhydrides of $\alpha$-$\beta$-unsaturated dicarboxylic acids such as maleic anhydride, itaconic anhydride and citraconic anhydride; and esters of acrylic acid or methacrylic acid with $C_{1-30}$ alkanols, such as ethyl acrylate, butyl acrylate, 2-ethylhexyl acrylate, dodecyl acrylate, hexadecyl acrylate, and octadecyl acrylate.

Optionally, other ethylenically unsaturated monomers can be copolymerized into the carboxylic acid polymer backbone. Example of such other ethylenically unsaturated monomers include styrene, vinyl acetate, ethylene, butadiene, acrylonitrile and mixtures thereof. Carboxylic acid polymers may preferably have a molecular weight of at least 1 million Daltons.

Suitable examples include crosslinked copolymers polymerized from $C_{1-4}$ alkyl acrylate or methacrylate (e.g. ethyl acrylate) with one or more comonomers selected from acrylic acid, methacrylic acid and mixtures thereof. Such materials may generally be referred to under the INCI name of Acrylates Copolymer. Commercially available examples include Aculyn® 33 from Rohm and Haas.

Also suitable are crosslinked copolymers polymerized from $C_{10-30}$ alkyl esters of acrylic or methacrylic acid with one or more comonomers selected from acrylic acid, methacrylic acid and their respective $C_{1-4}$ alkyl esters. Such materials may generally be referred to under the INCI name of Acrylates/C 10-30 Alkyl Acrylate Crosspolymer. Commercially available examples include Carbopol® polymers 1342 and 1382 from Lubrizol Advanced Materials.

Also suitable are optionally crosslinked copolymers of acrylic acid or methacrylic acid with alkyl acrylates and ethoxylated hydrophobically modified alkyl acrylates. Such materials may generally be referred to under the INCI names of Acrylates/Steareth-20 Methacrylate Copolymer, Acrylates/Beheneth-25 Methacrylate Copolymer, Acrylates/Steareth-20 Methacrylate Crosspolymer and Acrylates/Palmeth-25 Acrylates Copolymer. Commercially available examples include Aculyn® 22, 28 or 88 from Rohm & Haas and Synthalen® from 3V Sigma.

It is preferred that the carboxylic acid is a Carbomer, such as homopolymers of acrylic acid crosslinked with an allyl ether of pentaerythritol or an allyl ether of sucrose.

Mixtures of any of the aforementioned material may also be used.

Preferably haircare product of the invention comprises 0.1 to 3.0%, more preferably 0.4 to 1.5% carboxylic acid polymer by weight of the composition.

In formulations containing anionic polymeric rheology modifiers such as the carboxylic acid polymers described above, it is often necessary to neutralize at least a portion of the free carboxyl groups by the addition of an inorganic or organic base. Examples of suitable inorganic or organic bases include alkali metal hydroxides (e.g. sodium or potassium hydroxide), sodium carbonate, ammonium hydroxide, methylamine, diethylamine, trimethylamine, monoethanolamine, triethanolamine and mixtures thereof.

The haircare product of the invention may also comprise a nonionic polymeric rheology modifier which is selected from one or more nonionic cellulose ethers.

Suitable nonionic cellulose ethers or use as the nonionic polymeric rheology modifier in the invention include ($C_{1-3}$ alkyl) cellulose ethers, such as methyl cellulose and ethyl cellulose; hydroxy ($C_{1-3}$ alkyl) cellulose ethers, such as hydroxyethyl cellulose and hydroxypropyl cellulose; mixed hydroxy ($C_{1-3}$ alkyl) cellulose ethers, such as hydroxyethyl hydroxypropyl cellulose; and ($C_{1-3}$ alkyl) hydroxy ($C_{1-3}$ alkyl) cellulose ethers, such as hydroxyethyl methylcellulose and hydroxypropyl methylcellulose. Preferred nonionic cellulose ethers for use as the nonionic polymeric rheology modifier in the invention are water-soluble nonionic cellulose ethers such as methylcellulose and hydroxypropyl methylcellulose. The term "water-soluble" in this context denotes a solubility in water of at least 1 grams, more preferably at least 3 grams, most preferably at least 5 grams in 100 grams of distilled water at 25° C. and 1 atmosphere. This level indicates production of a macroscopically isotropic or transparent, coloured or colourless solution.

Methyl cellulose and hydroxypropyl methylcellulose are commercially available in a number of viscosity grades from Dow Chemical as their METHOCEL® trademark series.

Mixtures of any nonionic cellulose ethers may also be suitable. In a typical composition according to the invention the level of nonionic cellulose ethers will generally range from about 0.01 to about 2.0%, and preferably ranges from 0.1 to 0.5%, more preferably from 0.1 to 0.3%, by weight based on the total weight of the composition.

Preferably the haircare product of the invention comprises 0.1 to 0.3% by weight nonionic cellulose ether.

The haircare product of the invention may contain further optional ingredients to enhance performance and/or consumer acceptability. Examples of such ingredients include fragrance, dyes and pigments and preservatives. Each of these ingredients will be present in an amount effective to accomplish its purpose. Generally, these optional ingredients are included individually at a level of up to 5% by weight based on the total weight of the composition.

Mode of Use

The haircare product of the invention is primarily intended for topical application to hair and scalp.

When the haircare product is a shampoo, it is topically applied to the hair and then massaged into the hair and scalp. Then it is rinsed with water prior to drying the hair. A hair oil or hair serum, being leave-on haircare compositions, are left on for 1 to 10 hours after application before being washed off.

The invention will be further illustrated by the following, non-limiting Examples, in which all percentages quoted are by weight based on total weight unless otherwise stated.

The invention is not limited to the embodiments illustrated in the drawings. Accordingly, it should be understood that where features mentioned in the claims are followed by reference numerals, such numerals are included solely for the purpose of enhancing the intelligibility of the claims and are in no way limiting to the scope of the claims. The examples are intended to illustrate the invention and are not intended to limit the invention to those examples *per se.*

**Examples**

Example 1: Preparation of a composition (microparticles) outside the invention (Ref.1)

A gram of Octopirox® was dissolved in 12.5 g molten cetostearyl alcohol to form clear solution at 70°C. Thereafter 86.5 g DI water was taken in a 250 mL beaker and heated to 70°C with agitation. The Octopirox® solution in cetostearyl alcohol was added to the water under homogenizing at 2000 rpm for 15 minutes. The system was cooled gradually to room temperature under stirring. Then evaporated water was complemented until 100 g slurry was left behind (13.5 % solids content, i.e. microparticles). The particle size of the microparticles was 70 $\mu$m.

Example 2: Preparation of a composite inside the invention (Ref.2)

Three grams chitosan was dissolved in 300 mL of 0.5% aqueous acetic acid to form 1% chitosan solution. A gram of Octopirox® was dissolved in 12.5 g molten cetostearyl alcohol to form a clear solution at 70°C.

Thereafter 62.5 g of the 1% chitosan acetate solution and 24 g of the DI water were mixed in a beaker at 70°C. The Octopirox® solution was heated to 70°C and added to the contents of the beaker under homogenizing at 2000 rpm for 15 minutes. The system was cooled gradually to room temperature under stirring. Then evaporated water was complemented until 100 g slurry was left behind (14 % solids content, i.e. microparticles). The particle size of the microparticles was 54 $\mu$m.

Example 3: Preparation of a composite inside the invention (Ref.3)

A 1% solution of chitosan acetate was prepared as disclosed earlier. A gram of Octopirox® was dissolved in 12.5 g molten cetostearyl alcohol and 4 g damascone to form a clear solution at 70°C.

Thereafter 62.5 g of the 1% chitosan acetate solution and 20 g DI water were mixed in a beaker at 70°C. The Octopirox® solution was heated to 70°C and added to the contents of the beaker under homogenizing at 2000 rpm for 15 minutes. The system was cooled gradually to room temperature under stirring. Then evaporated water was complemented until 100 g aqueous slurry was left behind (18% solids content, i.e. microparticles).

The particle size of the microparticles was 43 $\mu$m.

Example 4: Preparation of a composite inside the invention (Ref.4)

A 1% solution of chitosan acetate was prepared as disclosed earlier. A gram of Octopirox® was dissolved in 8.5 g molten cetostearyl alcohol and 4 g damascone to form a clear solution at 70°C.

Thereafter 62.5 g of the 1% chitosan solution and 24 g DI water were mixed in a beaker 70°C. The Octopirox® solution was heated to 70°C and added to the beaker under homogenizing at 2000 rpm for 15 minutes. The system was cooled gradually to room temperature under stirring. Then evaporated water was complemented until 100 g slurry was left behind (14% solids content, i.e. microparticles). The particle size of the microparticles was 36 microns.

Details of the compositions of Examples 1 to 4 is summarised Table 1.

**Table 1**

| Example/wt% | Octopirox® | Cetostearyl alcohol | Damascone | Chitosan |
|---|---|---|---|---|
| 1 | 7.4 | 92.6 | -- | -- |

(continued)

| Example/wt% | Octopirox® | Cetostearyl alcohol | Damascone | Chitosan |
|---|---|---|---|---|
| 2 | 7.1 | 88.5 | -- | 4.4 |
| 3 | 5.5 | 69.0 | 22.1 | 3.4 |
| 4 | 7.1 | 60.2 | 28.3 | 4.4 |

The compositions of Examples 1 to 4 were subjected to a variety of tests which are described hereinafter.

Example 5: Deposition Efficacy

The extent of deposition (on scalp) of Octopirox® contained in the microparticles was evaluated by formulating a series of shampoo compositions 1A to 4A, each comprising a fixed amount of the microparticles from examples 1 to 4. In other words, shampoo 1A contained microparticles of Example 1 and so on.
The formulations are shown in Table 2.

**Table 2**

| Ingredients/wt% | Example Reference No. | | | | |
|---|---|---|---|---|---|
| | 1A | 2A | 3A | 4A | 5 |
| Octopirox® | - | - | - | - | 0.2 |
| Example 1 | 20 | - | - | - | - |
| Example 2 | - | 20 | - | - | - |
| Example 3 | - | - | 20 | - | - |
| Example 4 | - | - | - | 20 | - |
| SLES (70%) | 17.0 | | | | |
| CAPB (30%) | 5.0 | | | | |
| Sodium chloride | 1.0 | | | | |

The extent of deposition of Octopirox® on the scalp was determined as follows:
A piece of model skin (2cm x 2cm x 4mm) was placed in a Petridish®, to which 375 mg of the concerned shampoo (under evaluation) has been added. That piece and another blank piece (untreated) were then immersed in 10 ml methanol and dispersed with 10 minutes of ultrasonic treatment. An ml of the methanolic solution was passed through a 0.2 $\mu$m PTFE filter and transferred into Liquid Chromatography (LC) sample vial for LC analysis to get deposition amount data $D_0$.
A piece of model skin (2cm x 2cm x 4mm) was placed in a Petridish®, to which 375 mg of the concerned shampoo (under evaluation) has been added. That piece of shampoo on it was then gently rubbed with another piece (untreated) for 30 seconds and the two pieces were rinsed by 250 mL water. The rinsed pieces were then immersed in 10 ml methanol and dispersed with 10 minutes of ultrasonic treatment. An ml of the methanolic solution was passed through a 0.2 $\mu$m PTFE filter and transferred into Liquid Chromatography (LC) sample vial for LC analysis to get deposition amount data D.
Deposition efficacy was then calculated by following the equation:

$$\text{Octopirox deposition efficacy} = D / D_0 \times 100\%$$

The data is summarised in Table 3.

Table 3

| Sample Reference number | % Deposition |
|---|---|
| Example 5 | 1.9 |

(continued)

| Sample Reference number | % Deposition |
|---|---|
| Example 1A | 9.4 |
| Example 2A | 31.1 |
| Example 3A | 28.3 |
| Example 4A | 22.1 |

The data in Table 3 indicates that the % deposition provided by a composition (microparticles) outside the invention (Example 5 and 1A) is not as good as the extent of deposition that was observed in the case of Examples 2A-4A.

Example 6:

The objective of this experiment was to determine the extent of stability of Octopirox® contained in the microparticles of Examples 1 to 4 in a shampoo composition. It was evaluated by checking how much Octopirox® contained within the microparticles leaked into the shampoo base. For this test the shampoo products 5 and 1A to 4A were used immediately after preparation. The test was repeated after storing the compositions for 1 day, 7 days and 14 days.

For this experiment the shampoo products were made by mixing four grams of the slurry of the microparticles (Example 1 to 4 as the case may be) with 16 g of a shampoo base in 50 mL centrifuge tubes. Therefore, the formulations of the shampoos for the purpose of this experiment were different from the formulations (1A to 4A) disclosed in Table 2. To avoid confusion with nomenclature, the sample reference numbers used for this experiment were labelled as 1B to 4B.

Samples of the shampoos were stored at room temperature for a period of 1-day, 7-days and 14-days. At the predetermined time, 20 g deionized water was added into the centrifuge tube (containing 20 g shampoo) and the tubes were centrifuged at 10000 rpm for 20 minutes. Then the liquid part (supernatant) was collected for UV analysis to find out the concentration of Octopirox® in it which is represented as the % loss as compared to the amount of Octopirox® in a freshly prepared shampoo product.

The results are summarised in Table 4.

**Table 4**

| Sample Reference No. | Leakage/loss of Octopirox® | | |
|---|---|---|---|
| | 1-day | 7-day | 14-day |
| 1B | 90.0 | 100.0 | -- |
| 2B | 35.1 | 78.2 | 100.0 |
| 3B | 15.4 | 45.6 | 70.4 |
| 4B | 17.4 | 38.9 | 40.8 |

The data in Table 4 indicates that Octopirox was more stable in the case of compositions 2B to 4B. However, from among these three, the stability was the highest in the case of Example 4B. The microparticles of Example 4 contained a co-solvent (damascene) and this additional stability (or reduced leakage) can be attributed to damascone.

Example 7: Release of Octopirox® from microcapsules into sebum

For this experiment, the slurry of Example 4 was freeze-dried to get a powder. Seven (7) mg of this powder was incubated in 2 mL of a model sebum composition in an oven maintained at 32°C. At a predetermined time, the mixture was taken out of the oven and passed through a 0.2 μm PTFE filter to remove any insoluble matter. The filtrate was dispersed in methanol and transferred into a LC sample vial for LC analysis. Another 7 mg of the same powder was dissolved directly in methanol and the concentration of Octopirox® was measured by LC. This sample was for comparative purpose (control) and was for 100% release sample. Then the percentage of Octopirox® getting released from the composite was calculated.

The results showed that about 90% of the Octopirox® was released from the composite of Example 4 into sebum at 32°C. In other words, this observation indicates that a composite in accordance with the invention is stable enough inside the composition but at the same time, the composite is capable of releasing the Octopirox® when in contact with sebum, and this property renders it suitable for use in haircare products like a shampoo.

Example 8

A 1% solution of chitosan acetate was prepared as disclosed earlier. A gram of Octopirox® was dissolved in 8.5 g molten lauryl alcohol (MP 24°C) and 4 g damascone to form a clear solution at 40°C.
Thereafter 62.5 g of the chitosan acetate solution and 24 g DI water were measured in a beaker at 40°C. The Octopirox® solution was heated to 40°C and added to the beaker under homogenizing at 2000 rpm for 15 minutes. The system was cooled gradually to room temperature under stirring. Thereafter, the evaporated water was complemented to get 100 g of an aqueous slurry.
It was possible to prepare microparticles by this method but when the temperature increased above 24°C, the composite turned into a liquid indicating that the microparticles were not heat-stable.

Example 9

A 1% solution of chitosan acetate was prepared as disclosed earlier.
A gram of Octopirox® was dissolved in 8.5 g molten Fischer-Tropsch wax (Sasolwax® H1, MP 112°C) and 4 g damascone to form a clear solution at 120°C.
Thereafter 62.5 g of the 1% chitosan solution and 20 g DI water were measured in a beaker at 100°C. The Octopirox® solution was heated to 120°C and added to the beaker under homogenizing at 2000 rpm. The Octopirox® solution solidified immediately and failed to form fine composite particles.

**Claims**

1.  A composite particle comprising:

    (i) an antidandruff agent;
    (ii) a wax or a wax-like substance in which said antidandruff agent is soluble or dispersible, where melting point of said wax and said substance is 30°C to 105°C; and,
    (iii) a cationic polymer having weight average molecular weight of $10^3$ Da to $10^7$ Da,

    where said composite particle has particle size 0.1 to 1000 $\mu$m and where said wax-like substance is a C13 to 35 fatty alcohol; where said wax is at least one of beeswax, Chinese wax, lanolin, shellac wax, spermaceti, bayberry wax, candelilla wax, carnauba wax, castor wax, esparto wax, Japan wax, ouricury wax, rice bran wax, soy wax, tallow tree wax, ceresin wax, montan wax, ozocerite or peat wax.

2.  A composite particle as claimed in claim 1 wherein said composite particle comprises 0.1 to 80 wt% antidandruff agent, 18 to 95 wt% of the wax or wax-like substance and 0.5 to 10 wt% cationic polymer.

3.  A composite particle as claimed in claim 1 or 2 further comprising a co-solvent in which said antidandruff agent is soluble or dispersible, where said co-solvent is a ketone and where said co-solvent is neither wax nor a wax-like substance.

4.  A composite particle as claimed in claim 3 wherein said co-solvent is a ketone selected from 2-hexanone, 2-octanone or damascone.

5.  A composite particle as claimed in claim 3 or 4 wherein said composite particle comprises 0.2 to 30 wt% of said co-solvent.

6.  A composite particle as claimed in any of claims 1 to 5 wherein zeta potential of said cationic polymer is from +10 to +100 mV.

7.  A composite particle as claimed in any of claims 1 to 6 wherein said cationic polymer is one or more of polyamine, polyvinylpyrrolidone, polylysine, protamine, trimethylammonioethyl (meth)acrylate homopolymers and copolymers, acrylamidopropyl trimethylammonium halide homopolymers and copolymers, dialkyldiallylammonium halide homopolymers and copolymers, chitosan or derivatized chitosan, cellulose derivatives comprising trimethyl ammonium substituted epoxide, starch hydroxypropyl trimethyl ammonium halide, polyethyleneimines or polycondensates containing diquatemary ammonium or polyquatemary ammonium repeating units.

8. A composite particle as claimed in any of claims 1 to 7 wherein said antidandruff agent is piroctone olamine.

9. A composite particle as claimed in any of claims 1 to 8 wherein said composite particle is powdery.

10. A method of preparing a composite particle as claimed in claim 1 comprising a step of heating and agitating an aqueous slurry comprising said wax or wax-like substance and said antidandruff agent, followed by a step of adding said cationic polymer to said slurry and further heating said slurry for 5 to 60 minutes at 30 to 100 °C to get the composite particle or a slurry of the composite particle.

11. A haircare product comprising a composite particle as claimed in any of claims 1 to 9.

12. A haircare product as claimed in claim 11 wherein said haircare product comprises an amount of said composite particle as claimed in any of claims 1 to 9 such that the total amount of the antidandruff agent in said haircare product is from 0.01 to 5.0 wt%.

**Revendications**

1. Particule composite comprenant:

   (i) un agent antipelliculaire;
   (ii) une cire ou une substance semblable à de la cire dans laquelle ledit agent antipelliculaire est soluble ou peut être dispersé, où le point de fusion de ladite cire et de ladite substance est de 30°C à 105°C; et,
   (iii) un polymère cationique ayant un poids moléculaire moyen en poids de $10^3$ Da à $10^7$ Da, où ladite particule composite a une taille de particule de 0,1 à 1 000 $\mu$m et où ladite substance semblable à de la cire est un alcool gras en C13 à 35; où ladite cire est au moins l'une de cire d'abeille, cire de Chine, lanoline, cire de gomme-laque, spermacéti, cire de baie, cire de candelilla, cire de camauba, cire de ricin, cire d'alfa, cire du Japon, cire d'ouricury, cire de son de riz, cire de soja, cire d'arbre à suif, cire de cérésine, cire minérale, ozocérite ou cire de tourbe.

2. Particule composite selon la revendication 1, où ladite particule composite comprend 0,1 à 80 % en poids d'agent antipelliculaire, 18 à 95 % en poids de cire ou de substance semblable à de la cire et 0,5 à 10 % en poids de polymère cationique.

3. Particule composite selon la revendication 1 ou 2, comprenant en outre un co-solvant dans lequel ledit agent antipelliculaire est soluble ou peut être dispersé, où ledit co-solvant est une cétone et où ledit co-solvant n'est ni une cire ni une substance semblable à de la cire.

4. Particule composite selon la revendication 3, où ledit co-solvant est une cétone choisie parmi la 2-hexanone, la 2-octanone ou la damascone.

5. Particule composite selon la revendication 3 ou 4, où ladite particule composite comprend 0,2 à 30 % en poids dudit co-solvant.

6. Particule composite selon l'une quelconque des revendications 1 à 5, où le potentiel zêta dudit polymère cationique est de +10 à +100 mV.

7. Particule composite selon l'une quelconque des revendications 1 à 6, où ledit polymère cationique est un ou plusieurs de polyamine, polyvinylpyrolidone, polylysine, protamine, homopolymères et copolymères de (méth)acrylate de triméthylammonioéthyle, homopolymères et copolymères d'halogénures d'acrylamidopropyl triméthylammonium, homopolymères et copolymères d'halogénures de dialkyldiallylammonium, chitosane ou chitosane transformé en dérivé, dérivés de cellulose comprenant un époxyde substitué par du triméthylammonium, halogénure d'hydroxypropyltriméthylammonium d'amidon, polyéthylèneimines ou polycondensats contenant des unités répétées d'ammonium diquaternaire ou d'ammonium polyquaternaire.

8. Particule composite selon l'une quelconque des revendications 1 à 7, où ledit agent antipelliculaire est la piroctone olamine.

9. Particule composite selon l'une quelconque des revendications 1 à 8, où ladite particule composite est pulvérulente.

10. Procédé de préparation d'une particule composite selon la revendication 1 comprenant une étape de chauffage et d'agitation d'une suspension aqueuse comprenant ladite cire ou substance semblable à de la cire et ledit agent antipelliculaire, suivie d'une étape d'ajout dudit polymère cationique à ladite suspension et de chauffage supplémentaire de ladite suspension pendant 5 à 60 minutes à 30 à 100°C pour obtenir la particule composite ou une suspension de la particule composite.

11. Produit de soins capillaires comprenant une particule composite selon l'une quelconque des revendications 1 à 9.

12. Produit de soins capillaires selon la revendication 11, où ledit produit de soins capillaires comprend une quantité de ladite particule composite selon l'une quelconque des revendications 1 à 9 telle que la quantité totale de l'agent antipelliculaire dans ledit produit de soins capillaires est de 0,01 à 5,0 % en poids.


**Patentansprüche**

1. Kompositteilchen, umfassend:

(i) ein Antischuppenmittel;
(ii) ein Wachs oder eine wachsartige Substanz, in der das Antischuppenmittel löslich oder dispergierbar ist, wobei der Schmelzpunkt des Wachses und der besagten Substanz 30°C bis 105°C beträgt; und
(iii) ein kationisches Polymer mit einem gewichtsmittleren Molekulargewicht von $10^3$ Da bis $10^7$ Da,

wobei das Kompositteilchen eine Teilchengröße von 0,1 bis 1000 $\mu$m aufweist und wobei die wachsartige Substanz ein C13- bis C35-Fettalkohol ist; wobei das Wachs mindestens eines der folgenden Wachse ist: Bienenwachs, Chinawachs, Lanolin, Schellackwachs, Walrat, Lorbeerwachs, Candelillawachs, Carnaubawachs, Rizinuswachs, Espartowachs, Japanwachs, Ouricurywachs, Reiskleiewachs, Sojawachs, Talgbaumwachs, Ceresinwachs, Montanwachs, Ozokerit- oder Torfwachs.

2. Kompositteilchen, wie im Anspruch 1 beansprucht, wobei das Kompositteilchen 0,1 bis 80 Gew.-% Antischuppenmittel, 18 bis 95 Gew.-% Wachs oder wachsartige Substanz und 0,5 bis 10 Gew.-% kationisches Polymer umfasst.

3. Kompositteilchen, wie im Anspruch 1 oder 2 beansprucht, das außerdem ein Co-Lösungsmittel umfasst, in dem das Antischuppenmittel löslich oder dispergierbar ist, wobei das Co-Lösungsmittel ein Keton ist und wobei das Co-Lösungsmittel weder Wachs noch eine wachsartige Substanz ist.

4. Kompositteilchen, wie im Anspruch 3 beansprucht, wobei das Co-Lösungsmittel ein unter 2-Hexanon, 2-Octanon oder Damascon ausgewähltes Keton ist.

5. Kompositteilchen, wie im Anspruch 3 oder 4 beansprucht, wobei das Kompositteilchen 0,2 bis 30 Gew.-% Co-Lösungsmittel umfasst.

6. Kompositteilchen, wie in einem der Ansprüche 1 bis 5 beansprucht, wobei das Zetapotential des kationischen Polymers +10 bis +100 mV beträgt.

7. Kompositteilchen, wie in einem der Ansprüche 1 bis 6 beansprucht, wobei das kationische Polymer ein Polymer oder mehrere Polymere von folgenden ist: Polyamin, Polyvinylpyrrolidon, Polylysin, Protamin, Trimethylammonioethyl(meth)acrylat-Homopolymere und -Copolymere, Acrylamidopropyltrimethylammoniumhalogenid-Homopolymere und -Copolymere, Dialkyldiallylammoniumhalogenid-Homopolymere und -Copolymere, Chitosan oder derivatisiertes Chitosan, Cellulosederivate, umfassend Trimethylammoniumsubstituiertes Epoxid, Stärkehydroxypropyltrimethylammoniumhalogenid, Polyethylenimine oder Polycondensate, die sich wiederholende diquaternäre Ammonium- oder sich wiederholende polyquaternäre Ammonium-Einheiten enthalten.

8. Kompositteilchen, wie in einem der Ansprüche 1 bis 7 beansprucht, wobei das Antischuppenmittel Piroctonolamin ist.

**9.** Kompositteilchen, wie in einem der Ansprüche 1 bis 8 beansprucht, wobei das Kompositteilchen pulverförmig ist.

**10.** Verfahren zur Herstellung eines Kompositteilchens, wie im Anspruch 1 beansprucht, umfassend einen Schritt des Erhitzens und Rührens einer wässrigen Aufschlämmung, die das Wachs oder eine wachsartige Substanz und das Antischuppenmittel enthält, gefolgt von einem Schritt des Zugebens des kationischen Polymers zu der Aufschlämmung und weiterem Erhitzen der Aufschlämmung für 5 bis 60 Minuten bei 30 bis 100°C, um das Kompositteilchen oder eine Aufschlämmung des Kompositteilchens zu erhalten.

**11.** Haarpflegeprodukt, umfassend ein Kompositteilchen, wie in einem der Ansprüche 1 bis 9 beansprucht.

**12.** Haarpflegeprodukt, wie im Anspruch 11 beansprucht, wobei das Haarpflegeprodukt eine Menge des Kompositteilchens, wie in einem der Ansprüche 1 bis 9 beansprucht, umfasst, so dass die Gesamtmenge des Antischuppenmittels in dem Haarpflegeprodukt 0,01 bis 5,0 Gew.-% beträgt.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040213751 A1
- WO 14124066 A1
- WO 2008101546 A
- WO 10105922 A1
- EP 19154998 A
- WO 9823258 A1
- US 4009256 A
- WO 9522311 A

**Non-patent literature cited in the description**

- *CHEMICAL ABSTRACTS,* 131-57-7
- *CHEMICAL ABSTRACTS,* 131-53-3
- *CHEMICAL ABSTRACTS,* 70356-09-1
- *CHEMICAL ABSTRACTS,* 187393-00-6
- *CHEMICAL ABSTRACTS,* 134-09-8
- *CHEMICAL ABSTRACTS,* 36861-47-9
- *CHEMICAL ABSTRACTS,* 85-19-8
- *CHEMICAL ABSTRACTS,* 1641-17-4
- *CHEMICAL ABSTRACTS,* 1843-05-6